# EUROPÄISCHE PATENTSCHRIFT

(11) **EP 0 748 328 B1**
(45) Veröffentlichungstag und Bekanntmachung des Hinweises auf die Patenterteilung: **06.05.1999**
(21) Anmeldenummer: 95909767.6
(22) Anmeldetag: 22.02.1995
(51) Int. Cl.: C07H 13/06, C07H 15/12, A61K 7/48, C07C 233/18, C07C 233/56

(54) **PSEUDOCERAMIDE**
PSEUDOCERAMIDES
PSEUDOCERAMIDES

(30) Priorität: 03.03.1994 DE 4407016
(43) Veröffentlichungstag der Anmeldung: 18.12.1996
(73) Patentinhaber: Henkel Kommanditgesellschaft auf Aktien, 40191 Düsseldorf (DE)
(72) Erfinder: MÖLLER, Hinrich, D-40789 Monheim (DE); WACHTER, Rolf, D-40595 Düsseldorf (DE); BUSCH, Peter, D-40699 Erkrath (DE)
(86) Internationale Anmeldenummer: EP9500640
(87) Internationale Veröffentlichungsnummer: WO9523807

(56) Entgegenhaltungen:
- EP-A- 0 398 272
- EP-A- 0 450 527
- EP-A- 0 455 429
- DE-C- 4 238 210
- FR-A- 2 160 506
- CHEMICAL ABSTRACTS, vol. 92, no. 14, 7.April 1980 Columbus, Ohio, US; abstract no. 113228, ALIEV Z.E. ET AL 'Studies of the effect of some organic compounds on antifouling and anticorrosion properties of heavy fuels' Seite 139; Spalte 1; & CHEMICAL ABSTRACTS FORMULA INDEX 1977-1981, & PRISADKI SMAZ. MASLAM 1978, 5, 133-7,

## Beschreibung

### Gebiet der Erfindung

Die Erfindung betrifft Pseudoceramide, erhältlich durch Überführung von Alkylbernsteinsäuremonoestern bzw. -amiden in ihre Säurechloride bzw. gemischten Anhydride und anschließende Kondensation mit Aminverbindungen, ein Verfahren zur Herstellung der Pseudoceramide, Hautpflegemittel mit einem Gehalt der Pseudoceramide sowie die Verwendung der Pseudoceramide zur Herstellung von Hautpflegemitteln.

### Stand der Technik

Für die Elastizität und das Aussehen der Haut spielt ein ausbalancierter Wasserhaushalt in den einzelnen Hautschichten eine wichtige Rolle. In der Dermis und in der Grenzschicht der Epidermis nahe der Basalmembran ist der Gehalt an gebundenem Wasser am größten. Die Hautelastizität wird entscheidend durch die Collagenfibrillen in der Dermis geprägt, wobei die spezifische Konformation des Collagens durch den Einbau von Wassermolekülen erreicht wird. Eine Zerstörung der Lipidbarriere im Stratum Corneum (SC) beispielsweise durch Tenside führt zu einem Anstieg des transepidermalen Wasserverlustes, wodurch die wäßrige Umgebung der Zellen gestört wird. Da das in tieferen Hautschichten gebundene Wasser nur über Gefäße über die Körperflüssigkeit, nicht aber von außen zugeführt werden kann, wird deutlich, daß der Erhalt der Barrierefunktion des Stratum Corneum essentiell für den Gesamtzustand der Haut ist [vgl. S.E.Friberg et al., **C.R. 23. CED-Kongress, Barcelona, 1992, S.29**].

Ceramide stellen liphophile Amide langkettiger Fettsäuren dar, die sich im allgemeinen von Sphingosin bzw. Phytosphingosin ableiten. Erhebliche Bedeutung hat diese Klasse von körpereigenen Fettstoffen gewonnen, seitdem man sie im interzellären Raum zwischen den Corneozyten als Schlüsselkomponenten für den Aufbau des Lipid-Bilayers, also der Permeabilitätsbarriere, im Stratum Corneum der menschlichen Haut erkannt hat. Ceramide haben Molekulargewichte von deutlich unter 1000, so daß bei äußerer Zufuhr in einer kosmetischen Formulierung das Erreichen des Wirkortes möglich ist. Die externe Applikation von Ceramiden führt zur Restaurierung der Lipidbarriere, wodurch den geschilderten Störungen der Hautfunktion ursächlich entgegengewirkt werden kann [vgl. R.D. Petersen, **Cosm.Toil. 107, 45 (1992)**].

Dem Einsatz von Ceramiden sind infolge ihrer mangelnden Verfügbarkeit bislang Grenzen gesetzt. Es hat daher bereits Versuche gegeben, ceramidanaloge Strukturen, sogenannte "synthetic barrier lipids (SBL)" oder "Pseudoceramide" zu synthetisieren und zur Hautpflege einzusetzen [vgl. G.Imokawa et al. J.Soc. **Cosmet.Chem. 40, 273 (1989)**].

So werden beispielsweise in der Europäischen Offenlegungsschriften **EP-A 0 277 641** und **EP-A 0 227 994** (Kao) Ceramidanaloge der folgenden Struktur vorgeschlagen:

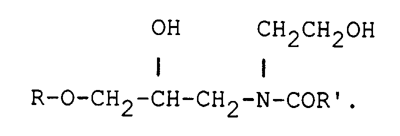

Aus den Europäischen Offenlegungsschriften **EP-A1 0 482 860** und **EP-A1 0 495 624** (Unilever) sind ceramidverwandte Strukturen der folgenden Formel bekannt:

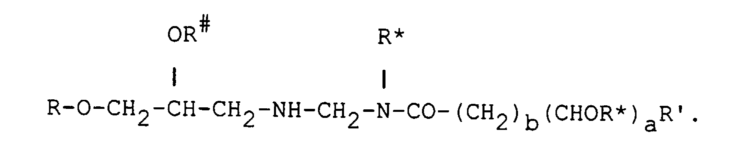

Für den Schutz von Haut und Haaren werden in der Europäischen Patentanmeldung **EP-A2 0 455 429** (Unilever) ferner Zuckerderivate der folgenden Zusammensetzung vorgeschlagen:

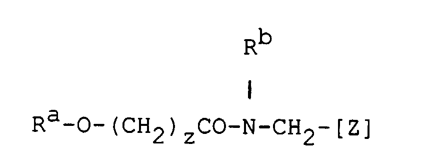

Hierbei steht R^{a} für Wasserstoff oder einen ungesättigten Fettacylrest, z für Zahlen von 7 bis 49, A für einen Hydroxyalkyl- und Z für einen Zucker- oder Phosphatrest.

Ungeachtet dieser Versuche ist der Erfolg, der sich mit diesen Stoffen erzielen läßt, bislang unbefriedigend; insbesondere wird das Leistungsvermögen natürlicher Ceramide nicht erreicht. Ferner sind die Synthesesequenzen technisch aufwendig und daher kostspielig, was die Bedeutung der Substanzen zusätzlich relativiert.

Die Aufgabe der Erfindung hat somit darin bestanden, neue leistungsstarke ceramidanaloge Strukturen zu entwickeln, die sich durch eine möglichst einfache Synthese auszeichnen. Eine weitere Aufgabe hat ferner darin bestanden, die neuen Pseudoceramide auf Basis nicht-tierischer Rohstoffe herzustellen.

### Beschreibung der Erfindung

Gegenstand der Erfindung sind Pseudoceramide der Formeln **(Ia)** bzw. **(Ib)**, 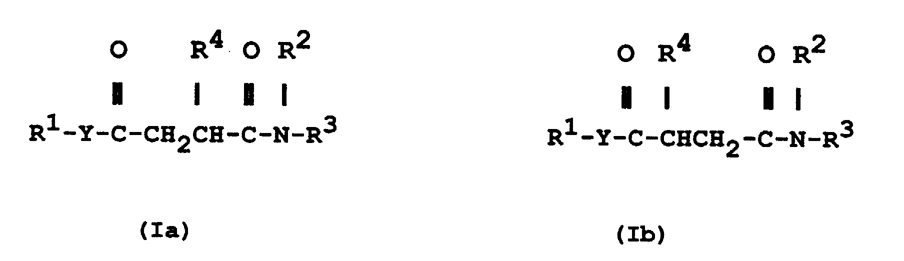 in der R¹ für einen linearen oder verzweigten Alkyl- und/oder Alkenylrest mit 6 bis 30 Kohlenstoffatomen, Y für Sauerstoff oder eine NR⁵-Gruppe, R² für Wasserstoff oder einen gegebenenfalls hydroxysubstituierten Alkylrest mit 1 bis 30 Kohlenstoffatomen, R³ für einen Hydroxyalkylrest mit 2 bis 12 Kohlenstoffatomen und 1 bis 10 Hydroxylgruppen oder einen Glycosylrest, R⁴ für einen Alkyl- und/oder Alkenylrest mit 6 bis 30 Kohlenstoffatomen und R⁵ für Wasserstoff oder einen gegebenenfalls hydroxysubstituierten Alkylrest mit 1 bis 6 Kohlenstoffatomen steht.

Überraschenderweise wurde gefunden, daß die im Sinne der Erfindung einzusetzenden Pseudoceramide die natürliche Barrierefunktion der Haut stärken, die Haut festigen und vor Austrocknung schützen. Die Stoffe sind den natürlichen Hautlipiden nachempfunden, dermatologisch und ökotoxikologisch unbedenklich und lassen sich homogen in die Ölphase kosmetischer Mittel einarbeiten. Sie sind weiß bzw. elfenbeinartig gefärbt, geruchsfrei, im Bereich des Haut-pH-Wertes hydrolysebeständig und farbstabil gegen Luftsauerstoff. Die Erfindung schließt die Erkenntnis ein, daß die Pseudoceramide auf Basis pflanzlicher Fettalkohole und Zukker, also ohne Mitverwendung unerwünschter tierischer Rohstoffe, hergestellt werden können.

Besonders bevorzugt sind Pseudoceramide der Formel **(I)** in der R¹ für einen Alkyl- und/oder Alkenylrest mit 12 bis 18 Kohlenstoffatomen, Y für Sauerstoff, R² für Wasserstoff oder eine Methylgruppe, R³ für einen Hydroxyalkylrest mit 6 Kohlenstoffatomen und 5 Hydroxylgruppen oder einen Glucosylrest und R⁴ für einen Alkyl- und/oder Alkenylrest mit 12 bis 18 Kohlenstoffatomen steht.

### Herstellverfahren

Ein weiterer Gegenstand der Erfindung betrifft ein Verfahren zur Herstellung von Pseudoceramiden der Formeln **(Ia)** bzw. **(Ib)**, 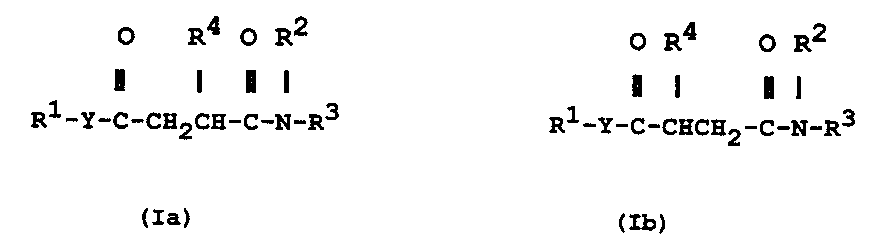 in der R¹ für einen linearen oder verzweigten Alkyl- und/oder Alkenylrest mit 6 bis 30 Kohlenstoffatomen, Y für Sauerstoff oder eine NR⁵-Gruppe, R² für Wasserstoff oder einen gegebenenfalls hydroxysubstituierten Alkylrest mit 1 bis 30 Kohlenstoffatomen, R³ für einen Hydroxyalkylrest mit 2 bis 12 Kohlenstoffatomen und 1 bis 10 Hydroxylgruppen oder einen Glycosylrest, R⁴ für einen Alkyl- und/oder Alkenylrest mit 6 bis 30 Kohlenstoffatomen und R⁵ für Wasserstoff oder einen gegebenenfalls hydroxysubstituierten Alkylrest mit 1 bis 6 Kohlenstoffatomen steht, daß sich dadurch auszeichnet, daß man
a) Alkylbernsteinsäuremonoester bzw. -amide der Formeln **(IIa)** bzw. **(IIb)**, 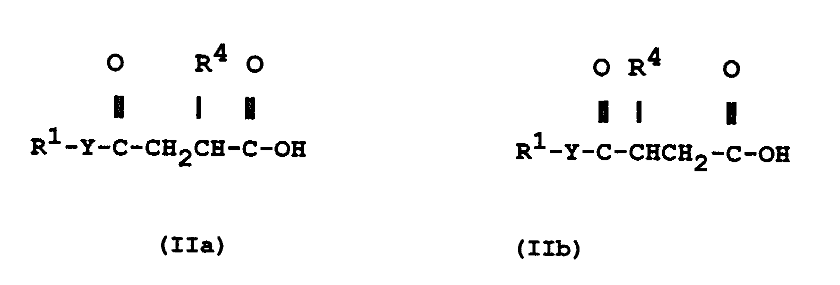 in der R¹, R⁴ und Y die oben genannten Bedeutungen besitzen, in an sich bekannter Weise mittels Chlorverbindungen in ihre Säurechloride oder gemischten Anhydride überführt und
b) die resultierenden Säurechloride bzw. gemischten Anhydride mit Hydroxyalkylaminen bzw. Glucosylaminen der Formel **(III)** kondensiert, 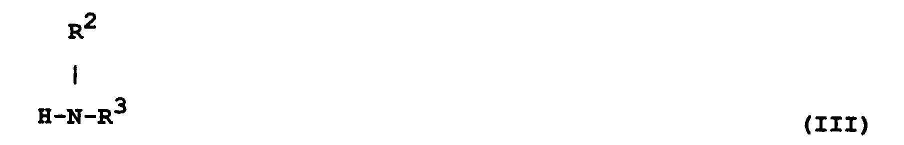 wobei R² und R³ wiederum die oben angegebenen Bedeutungen besitzen.

### Alkylbernsteinsäuremonoester bzw. -amide

Im Sinne des erfindungsgemäßen Verfahrens kommen Monoester bzw. Monoamide der Alkylbernsteinsäure in Betracht, die bekannte chemische Verbindungen darstellen. Vorzugsweise werden Alkylbernsteinsäuremonoester der Formel **(II)** eingesetzt, in der R¹ für einen Alkyl- und/oder Alkenylrest mit 12 bis 18 Kohlenstoffatomen und R⁴ für einen Alkyl- und/oder Alkenylrest mit 12 bis 18 Kohlenstoffatomen steht.

Typische Beispiele für Alkylbernsteinsäuremonoester sind Ester der Hexyl-, Octyl-, 2-Ethylhexyl-, Decyl-, Dodecyl-, Hexadecyl- und/oder Octadecylbernsteinsäure mit Capronalkohol, Caprylalkohol, 2-Ethylhexylalkohol, Caprinalkohol, Laurylalkohol, Isotridecylalkohol, Myristylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Arachylalkohol sowie deren technischen Gemischen wie sie beispielweise bei der Hochdruckhydrierung von technischen Fettsäuremethylesterfraktionen oder Aldehyden aus der roelen'schen Oxosynthese anfallen. Besonders bevorzugt sind Ester der Hexadecyl- und/oder Octadecylbernsteinsäure mit technischen C₁₂/₁₈-Kokos-, C_{16/18}-Palm- oder C_{16/18}-Talgfettalkoholschnitten.

Typische Beispiel für Alkylbernsteinsäuremonoamide sind Amide der Hexyl-, Octyl-, 2-Ethylhexyl-, Decyl-, Dodecyl-, Hexadecyl- und/oder Octadecylbernsteinsäure mit Hexylamin, Decylamin, Dodecylamin, Tetradecylamin, Hexadecylamin, Octadecylamin, Behenylamin, C_{16/18}-Palmfettalkylamin, C_{16/18}-Talgfett-alkylamin, N-Methyldodecylamin, N-Methyloctadecylamin, Ethanolamin und/oder Propanolamin.

### Chlorverbindungen

Um eine Verknüpfung zwischen den Acylierungsprodukten und den Hydroxyalkylaminen herstellen zu können, wird die freie Carboxylgruppe der Acylierungsprodukte zunächst in an sich bekannter Weise in das Säurechlorid überführt. Als Chlorverbindungen werden dabei vorzugsweise Phosphortrichlorid oder Thionylchlorid eingesetzt. Üblicherweise kann man die Acylierungsprodukte und die Chlorverbindungen im molaren Verhältnis 1 : 0,4 bis 1 : 2,5 einsetzen. Die Chlorierung wird vorzugsweise bei -10 bis 50°C in Abwesenheit von Wasser durchgeführt, als Lösungsmittel kommen beispielsweise Benzinfraktionen, Toluol, Ethylacetat, tert. Butylmethylether oder Tetrahydrofuran in Betracht. Um eine Wärmeabfuhr der stark exothermen Reaktion sicherzustellen, empfiehlt es sich, die Chlorierung zunächst im Eisbad durchzuführen. Nach Abschluß der Reaktion werden die Verunreinigungen, z.B. unterphosphorige Säure abgeschieden, nichtumgesetztes Chlorierungsmittel abdestilliert oder - wenn es sich nur um geringe Mengen handelt - in der Reaktionsmischung belassen.

In einer Variante des erfindungsgemäßen Verfahrens können die Acylierungsprodukte mit Chlorkohlensäurealkylestern in einem inerten Lösungsmittel zu den gemischten Anhydriden umgesetzt werden. Die Umsetzung findet vorzugsweise in Gegenwart eines Säurefängers wie Triethylamin, Tributylamin oder Natrium-bzw. Kaliumcarbonat statt, wobei die Reaktionspartner in etwa molaren Mengen bei Temperaturen von -10 bis 50, vorzugsweise 0 bis 10°C eingesetzt werden. Im Anschluß an die Umsetzung empfiehlt es sich, gebildete Salze abzufiltrieren. In diesem Zusammenhang sei auf die Veröffentlichung von C.Bersena in **J.Org.Chem. 27, 3489 (1962)** verwiesen. Für die nachfolgende Reaktion kann entweder die erhaltene Lösung oder aber deren Trockenrückstand eingesetzt werden.

### Hydroxyalkylamine

Als Hydroxyalkalyamine, die mit den Säurechloriden bzw. gemischten Anhydriden der Acylierungsprodukte zur Reaktion gebracht werden, kommen beispielsweise **N-Alkylglucosylamine** der Formel **(IV)** in Betracht, 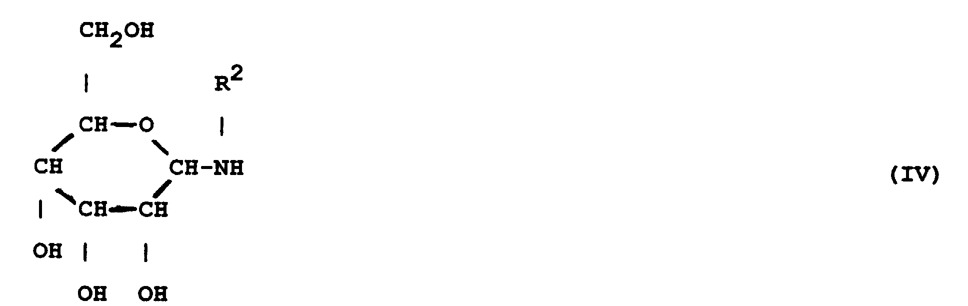 in der R² vorzugsweise für einen Alkylrest mit 1 bis 22 und insbesondere 1 bis 4 Kohlenstoffatomen steht.

In einer weiteren bevorzugten Ausführungsform der Erfindung werden N-Alkylsorbitylamine und vorzugsweise **N-Alkylglucamine** der Formel **(V)** eingesetzt, 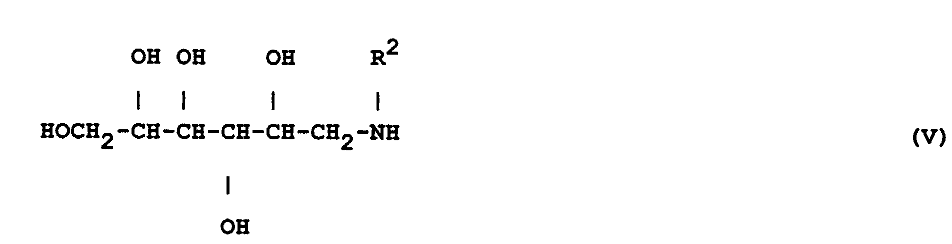 in der R² vorzugsweise für einen Alkylrest mit 1 bis 22 und insbesondere 1 bis 4 Kohlenstoffatomen steht. Die N-Alkylglucamine werden üblicherweise durch reduktive Aminierung von Glucose mit Fettaminen hergestellt. Daneben können sich sowohl die Glucosylamine als auch die Glucamine beispielsweise auch von Maltose, Fructose oder Palatinose ableiten. Als weitere Hydroxyalkylamine kommen ferner auch methylolsubstituierte Alkanolamine, beispielsweise Ethanolamin, Propanolamin, 1,1-Bis(hydroxymethyl)-2-aminoethanol, 2,2-Bis-(hydroxymethyl) -2-aminoethanol und 2,2-Bis-(hydroxymethyl)-3-aminopropanol in Betracht.

Üblicherweise kann man die Säurechloride bzw. gemischten Anhydride und die Hydroxyalkylamine bzw. Glucosylamine im molaren Verhältnis von 1 : 0,9 bis 1 : 1,1 einsetzen. Die Kondensationsreaktion wird vorzugsweise bei Temperaturen im Bereich von -10 bis 50°C in Gegenwart alkalischer Katalysatoren durchgeführt, wobei die Reaktionszeiten typischerweise 1 bis 10 h betragen können. Als Säurefänger können Soda, Pottasche oder tertiäre Amine wie z.B. Triethylamin eingesetzt werden.

Als Lösungsmittel empfiehlt sich beispielsweise Tetrahydrofuran. Im Anschluß können die Produkte durch Umkristallisation beispielsweise aus niederen Alkoholen oder Säulenchromatographie gereinigt werden. Die Kondensation von Aminverbindungen mit Säurechloriden ist grundsätzlich bekannt und wird beispielsweise in der **EP-A 0 265 818** (CF Stockhausen) beschrieben.

### Reaktionsschema

Demzufolge zeichnet sich das Herstellverfahren durch die Abfolge nachstehender - beispielhafter - Reaktionen aus, die dem Verständnis des Reaktionsgeschehens dienlich sein sollen:

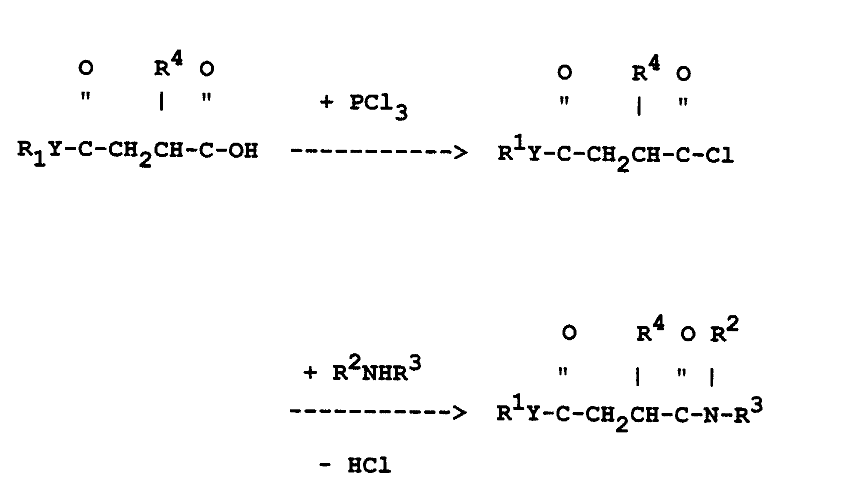

### Hautpflegemittel

Hautpflegemittel können die Pseudoceramide in Mengen von 1 bis 30, vorzugsweise von 2 bis 10 Gew.-% - bezogen auf die Mittel - enthalten und dabei sowohl als "Wasser-in-Öl" als auch "Öl-in-Wasser"-Emulsionen vorliegen; weitere übliche Hilfs- und Zusatzstoffe in Mengen von 5 bis 95, vorzugsweise 10 bis 80 Gew.-% können zudem enthalten sein. Ferner können die Formulierungen Wasser in einer Menge bis zu 99 Gew.-%, vorzugsweise 5 bis 80 Gew.-% aufweisen.

Als **Trägeröle** kommen hierzu beispielsweise in Betracht: Mineralöle, Pflanzenöle, Siliconöle, Fettsäureester, Dialkylether, Fettalkohole und Guerbetalkohole. Als **Emulgatoren** können beispielsweise eingesetzt werden: Sorbitanester, Monoglyceride, Polysorbate, Polyethylenglycolmono/difettsäureester, hochethoxylierte Fettsäureester sowie hochmolekulare Siliconverbindungen, wie z.B. Dimethylpolysiloxane mit einem durchschnittlichen Molekulargewicht von 10.000 bis 50.000. Weitere Zusatzstoffe können sein: **Konservierungsmittel**, wie z.B. p-Hydroxybenzoesäureester; **Antioxidantien**, wie z.B. Butylhydroxytoluol, Tocopherol; **Feuchthaltemittel**, wie z.B. Glycerin, Sorbitol, 2-Pyrrolidin-5-carboxylat, Dibutylphthalat, Gelatine, Polyglycole mit einem durchschnittlichen Molekulargewicht von 200 bis 600; **Puffer**, wie z.B. Milchsäure/TEA oder Milchsäure/NaOH; **milde Tenside**, wie z.B. Alkyloligoglucoside, Fettalkoholethersulfate, Fettsäureisethionate, -tauride und - sarcosinate, Ethercarbonsäuren, Sulfosuccinate, Eiweißhydrolysate bzw. -fettsäurekondensate, Sulfotriglyceride, kurzkettige Glucamide; **Phospholipide, Wachse**, wie z.B. Bienenwachs, Ozokeritwachs, Paraffinwachs; **Pflanzenextrakte**, z.B. von Aloe vera; **Verdickungsmittel**; **Farb- und Perfümstoffe** sowie **Sonnenschutzmittel**, wie z.B. ultrafeines Titandioxid oder organische Stoffe wie p-Aminobenzoesäure und deren Ester, Ethylhexyl-p-methoxyzimtsäureester, 2-Ethoxyethyl-p-methoxyzimtsäureester, Butylmethoxydibenzoylmethan und deren Mischungen.

In einer bevorzugten Ausführungsform der Erfindung können die Pseudoceramide mit konventionellen Ceramiden, weiteren Pseudoceramiden, Cholesterin, Cholesterinfettsäureestern, Fettsäuren, Triglyceriden, Cerebrosiden, Phospholipiden und ähnlichen Stoffen, abgemischt werden, wobei Liposomen entstehen können.

In einer weiteren bevorzugten Ausführungsform der Erfindung können die Pseudoceramide mit Wirkstoffbeschleunigern, insbesondere mit etherischen Ölen, wie beispielsweise Eucalyptol, Menthol und ähnlichen abgemischt werden.

In einer dritten bevorzugten Ausführungsform können die Pseudoceramide schließlich auch in Squalen oder Squalan gelöst und gegebenenfalls mit den anderen genannten Inhaltsstoffen zusammen mit flüchtigen oder nichtflüchtigen Siliconverbindungen als wasserfreie oder beinahe wasserfreie einphasige Systeme formuliert werden. Weitere Beispiele zu Bestandteilen und typischen Zusammensetzungen können beispielsweise der **WO 90/01323** (Bernstein) entnommen werden.

### Gewerbliche Anwendbarkeit

Die im Sinne der Erfindung als "synthetic barrier lipids" einzusetzenden Pseudoceramide stärken die natürliche Barrierefunktion der Haut gegenüber äußeren Reizen. Sie verbessern Festigkeit, Geschmeidigkeit und Elastizität der Haut, steigern den Feuchtigkeitsgehalt und schützen die Haut vor Austrocknung; zugleich werden feinste Falten geglättet.

Ein weiterer Gegenstand der Erfindung betrifft daher die Verwendung von Pseudoceramiden der Formel **(I)** als "synthetic barrier lipids" zur Herstellung von Hautpflegemitteln, in denen sie in Mengen von 1 bis 30, vorzugsweise 2 bis 10 Gew.-% - bezogen auf die Mittel - enthalten sein können. Typische Beispiele sind Hautcremes, Softcremes, Nährcremes, Sonnenschutzcremes, Nachtcremes, Hautöle, Pflegelotionen und Körper-Aerosole.

Die folgenden Beispiele sollen den Gegenstand der Erfindung näher erläutern, ohne ihn darauf einzuschränken.

### Beispiele

### Beispiel 1:

**Hexadeylbernsteinsäure-behenylester-N-methylsorbitylamid.** Zu der gerührten Lösung von 65,1 g (0,1 mol) Hexadecylbernsteinsäuremonobehenylester in 150 ml Benzin (Sdp. 65 bis 90°C) wurde bei Raumtemperatur eine Lösung von 27,4 g (0,2 mol) Phosphortrichlorid in 30 ml Benzin gegeben und die Mischung 1 h bei dieser Temperatur und danach 3 h bei 40°C gerührt. Nach dem Absetzen der öligen Phosphorigen Säure wurde davon abdekantiert, die Lösung einrotiert, der farblose wachsartige Rückstand in 250 ml tert.Butylmethylether gelöst, die Lösung bei 10°C unter Rühren mit einer Lösung von 21,5 g (0,11 mol) N-Methylsorbitylamin und 41,5 g (0,23 mol) Kaliumcarbonat in 70 ml Wasser langsam versetzt. Die entstandene steife Emulsion erwärmte sich auf 15°C und wurde nach Zugabe von 100 ml tert.Butylmethylether bei Raumtemperatur über Nacht gerührt. Anschließend wurde 1 bis 2 h auf 50°C erwärmt, wobei Phasentrennung eintrat, die organische Phase abgetrennt, die wäßrige Phase dreimal mit je 100 ml tert. Butylmethylether gewaschen und die vereinigten organischen Phasen eingedampft. Es wurden 65,3 g (entsprechend 79 % der Theorie) eines farblosen Wachses erhalten, das bei 47 bis 50°C sinterte und bei 140°C allmählich glasartig klar wurde.

### Beispiel 2:

**Hexadecylbernsteinsäure-behenylester-1,1-bishydroxymethyl-2-heydroxyethylamid.** Analog Beispiel 1 wurden 65,1 g (0,1 mol) Hexadecylbernsteinsäuremonobehenylester und 0,11 mol 1,1-Bishydroxymethyl-2-hydroxyethylamin umgesetzt. Es wurde ein farbloses Wachs erhalten, das bei 45°C sinterte und bei 120°C langsam schmolz.

### Beispiel 3:

Zu der gerührten Lösung von 59,5 g (0,1 mol) Hexadecylbernsteinsäuremonooctadecylester und 11,2 g (0,115 mol) Triethylamin in 250 ml THF wurde bei -5°C eine Lösung von 12,5 Chlorkohlensäureethylester in 50 ml THF gegeben und die Mischung 2 h bei 0°C weiter gerührt, wobei Triethylaminhydrochlorid ausfiel. Anschließend wurde bei 10°C zu der gerührten Suspension eine Mischung von 13,3 g (0,11 mol) 1,1-Bishydroxymethyl-2-hydroxyethylamin in 100 ml THF gegeben, 2 h bei 10°C, 3 h bei 22°C und 2 h bei 40°C weiter gerührt. Nach Abfiltrieren des Triethylaminhydrochlorids, Eindampfen der Lösung, Digerieren des Rückstandes mit Isopropylalkohol und Trocknen des Filterrückstands wurden 64,9 g (entsprechend 93 % der Theorie) eines farblosen Wachses erhalten, das bei 42°C sinterte und allmählich bis 115°C schmolz.

## Patentansprüche

1. Pseudoceramide der Formeln **(Ia)** bzw. **(Ib)**, 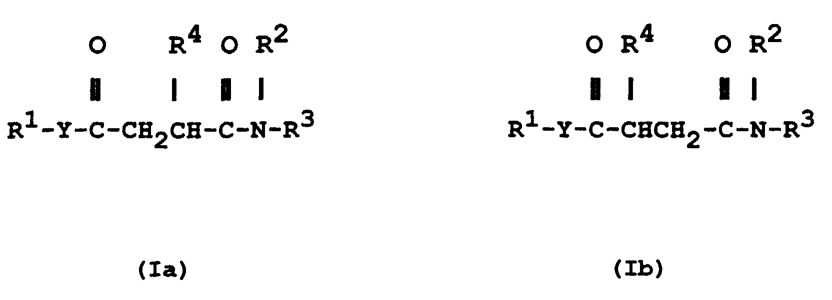 in der R¹ für einen linearen oder verzweigten Alkyl- und/oder Alkenylrest mit 6 bis 30 Kohlenstoffatomen, Y für Sauerstoff oder eine NR⁵-Gruppe, R² für Wasserstoff oder einen gegebenenfalls hydroxysubstituierten Alkylrest mit 1 bis 30 Kohlenstoffatomen, R³ für einen Hydroxyalkylrest mit 2 bis 12 Kohlenstoffatomen und 1 bis 10 Hydroxylgruppen oder einen Glycosylrest, R⁴ für einen Alkyl- und/oder Alkenylrest mit 6 bis 30 Kohlenstoffatomen und R⁵ für Wasserstoff oder einen gegebenenfalls hydroxysubstituierten Alkylrest mit 1 bis 6 Kohlenstoffatomen steht.

2. Pseudoceramide nach Anspruch 1, **dadurch gekennzeichnet**, daß in Formel **(I)** R¹ für einen Alkyl- und/oder Alkenylrest mit 12 bis 18 Kohlenstoffatomen, Y für Sauerstoff, R² für Wasserstoff oder eine Methylgruppe, R³ für einen Hydroxyalkylrest mit 6 Kohlenstoffatomen und 5 Hydroxylgruppen oder einen Glucosylrest und R⁴ für einen Alkyl- und/oder Alkenylrest mit 12 bis 18 Kohlenstoffatomen steht.

3. Verfahren zur Herstellung von Pseudoceramiden der For meln **(Ia)** bzw. **(Ib)**, 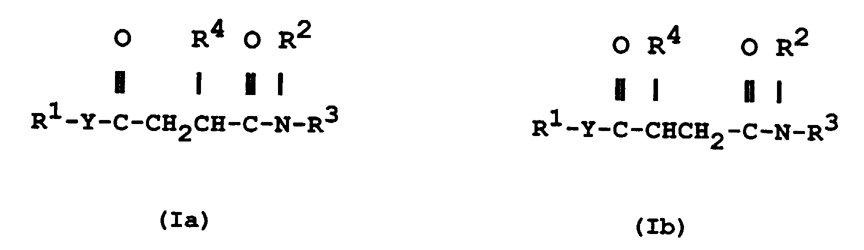 in der R¹ für einen linearen oder verzweigten Alkyl- und/oder Alkenylrest mit 6 bis 30 Kohlenstoffatomen, Y für Sauerstoff oder eine NR⁵-Gruppe, R² für Wasserstoff oder einen gegebenenfalls hydroxysubstituierten Alkylrest mit 1 bis 30 Kohlenstoffatomen, R³ für einen Hydroxyalkylrest mit 2 bis 12 Kohlenstoffatomen und 1 bis 10 Hydroxylgruppen oder einen Glycosylrest, R⁴ für einen Alkyl- und/oder Alkenylrest mit 6 bis 30 Kohlenstoffatomen und R⁵ für Wasserstoff oder einen gegebenenfalls hydroxysubstituierten Alkylrest mit 1 bis 6 Kohlenstoffatomen steht, **dadurch gekennzeichnet**, daß man
a) Alkylbernsteinsäuremonoester bzw. -amide der Formeln **(IIa)** bzw. **(IIb)**, 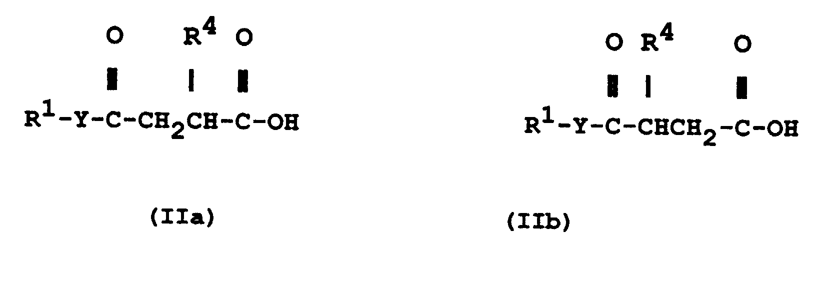 in der R¹, R⁴ und Y die oben genannten Bedeutungen besitzen, in an sich bekannter Weise mittels Chlorverbindungen in ihre Säurechloride bzw. gemischten Anhydride überführt und
b) die resultierenden Säurechloride bzw. gemischten Anhydride mit Hydroxyalkylaminen bzw. Glucosylaminen der Formel **(III)** kondensiert, 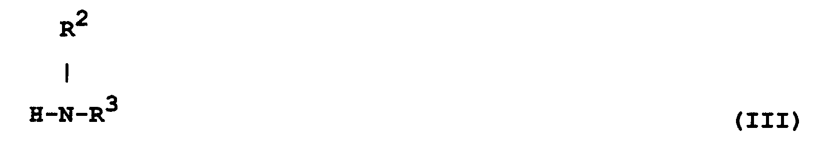 wobei R² und R³ wiederum die oben angegebenen Bedeutungen besitzen.

4. Verfahren nach Anspruch 3, **dadurch gekennzeichnet**, daß man Alkylbernsteinsäuremonoester der Formel **(II)** einsetzt, in der R¹ für einen Alkyl- und/oder Alkenylrest mit 12 bis 18 Kohlenstoffatomen und R⁴ für einen Alkyl- und/oder Alkenylrest mit 12 bis 18 Kohlenstoffatomen steht.

5. Verfahren nach den Ansprüchen 3 und 4, **dadurch gekennzeichnet**, daß man als Chlorverbindungen Phosphortrichlorid, Thionylchlorid oder Chlorkohlensäureester einsetzt.

6. Verfahren nach den Ansprüchen 3 bis 5, **dadurch gekennzeichnet**, daß man die Alkylbernsteinsäuremonoester bzw. -amide und die Chlorverbindungen im molaren Verhältnis 1 : 0,9 bis 1 : 1,1 einsetzt.

7. Verfahren nach den Ansprüchen 3 bis 6, **dadurch gekennzeichnet**, daß man als Hydroxyalkylamine Glucosylamine, Glucamine oder 2,2-Bis(hydroxymethyl)-2-aminoethanol einsetzt.

8. Verfahren nach den Ansprüchen 3 bis 7, **dadurch gekennzeichnet**, daß man die Säurechloride bzw. gemischten Anhydride und die Hydroxyalkylamine bzw. Glucosylamine im molaren Verhältnis 1 : 0,9 bis 1 : 1,1 einsetzt.

9. Verwendung von Pseudoceramiden nach Anspruch 1 als "synthetic barrier lipids" zur Herstellung von Hautpflegemitteln.

## Claims

1. Pseudoceramides corresponding to formulae **(Ia)** and **(Ib)**: 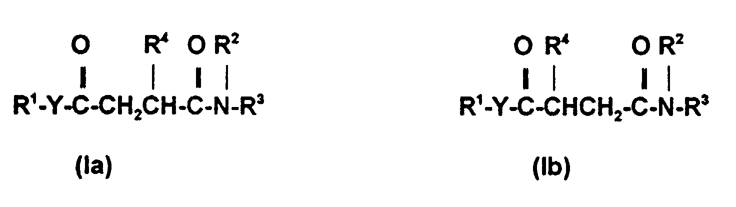 in which R¹ is a linear or branched alkyl and/or alkenyl radical containing 6 to 30 carbon atoms, Y is oxygen or an NR⁵ group, R² is hydrogen or an optionally hydroxysubstituted alkyl radical containing 1 to 30 carbon atoms, R³ is a hydroxyalkyl radical containing 2 to 12 carbon atoms and 1 to 10 hydroxyl groups or a glycosyl radical, R⁴ is an alkyl and/or alkenyl radical containing 6 to 30 carbon atoms and R⁵ is hydrogen or an optionally hydroxysubstituted alkyl radical containing 1 to 6 carbon atoms.

2. Pseudoceramides as claimed in claim 1, characterized in that in formula (I), R¹ is an alkyl and/or alkenyl radical containing 12 to 18 carbon atoms, Y is oxygen, R² is hydrogen or a methyl group, R³ is a hydroxyalkyl radical containing 6 carbon atoms and 5 hydroxyl groups or a glucosyl radical and R⁴ is an alkyl and/or alkenyl radical containing 12 to 18 carbon atoms.

3. A process for the production of pseudoceramides corresponding to formulae **(Ia)** and **(Ib)**: 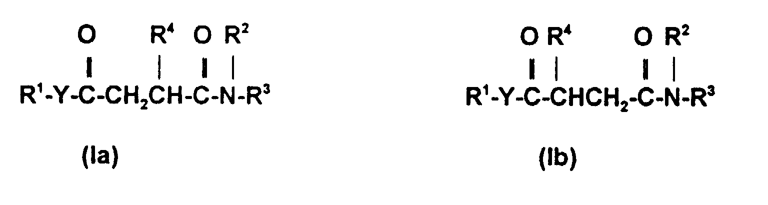 in which R¹ is a linear or branched alkyl and/or alkenyl radical containing 6 to 30 carbon atoms, Y is oxygen or an NR⁵ group, R² is hydrogen or an optionally hydroxysubstituted alkyl radical containing 1 to 30 carbon atoms, R³ is a hydroxyalkyl radical containing 2 to 12 carbon atoms and 1 to 10 hydroxyl groups or a glycosyl radical, R⁴ is an alkyl and/or alkenyl radical containing 6 to 30 carbon atoms and R⁵ is hydrogen or an optionally hydroxysubstituted alkyl radical containing 1 to 6 carbon atoms, characterized in that
a) alkyl succinic acid monoesters or amides corresponding to formula **(IIa)** or **(IIb)**: 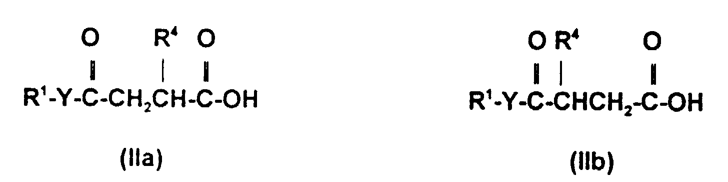 in which R¹ , R⁴ and Y are as defined above,
are converted into their acid chlorides or mixed anhydrides in known manner using chlorine compounds and
b) the resulting acid chlorides or mixed anhydrides are condensed with hydroxyalkylamines or glucosylamines corresponding to formula **(III)**: 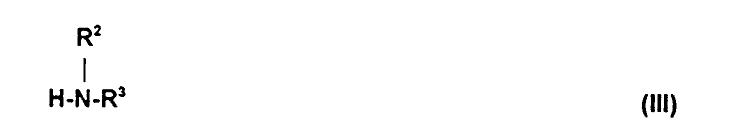 in which R² and R³ are again as defined above.

4. A process as claimed in claim 3, characterized in that alkyl succinic acid monoesters corresponding to formula (II), in which R¹ is an alkyl and/or alkenyl radical containing 12 to 18 carbon atoms and R⁴ is an alkyl and/or alkenyl radical containing 12 to 18 carbon atoms, are used.

5. A process as claimed in claims 3 and 4, characterized in that phosphorus trichloride, thionyl chloride or chlorocarbonic acid esters are used as the chlorine compounds.

6. A process as claimed in claims 3 to 5, characterized in that the alkyl succinic acid monoesters or amides and the chlorine compounds are used in a molar ratio of 1:0.9 to 1:1.1.

7. A process as claimed in claims 3 to 6, characterized in that glucosylamines, glucamines or 2,2-bis-(hydroxymethyl)-2-aminoethanol are used as the hydroxyalkylamines.

8. A process as claimed in claims 3 to 7, characterized in that the acid chlorides or mixed anhydrides and the hydroxyalkylamines or glycosylamines are used in a molar ratio of 1:0.9 to 1:1.1.

9. The use of the pseudoceramides claimed in claim 1 as synthetic barrier lipids for the production of skin-care formulations.

## Revendications

1. Pseudocéramides des formules (Ia) ou (Ib) : 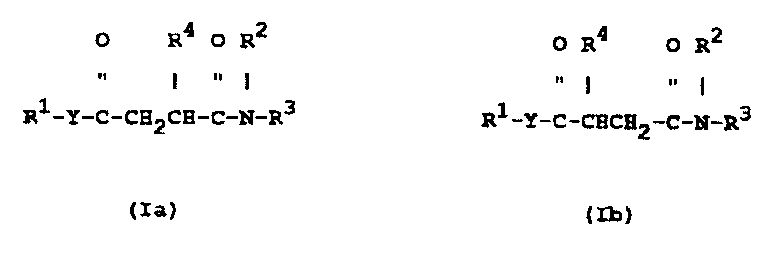 dans lesquelles R¹ représente un radical alkyle et/ou alkényle ayant de 6 à 30 atomes de carbone, linéaire ou ramifié, Y représente de l'oxygène ou un groupe NR⁵, R² représente de l'hydrogène ou un radical alkyle éventuellement substitué par un hydroxy, ayant de 1 à 30 atomes de carbone, R³ représente un radical hydroxyalkyle ayant de 2 à 12 atomes de carbone et de 1 à 10 groupes hydroxyle ou un reste glycosyle, R⁴ représente un radical alkyle et/ou alkényle ayant de 6 à 30 atomes de carbone et R⁵ représente de l'hydrogène ou un radical alkyle éventuellement substitué par un hydroxy, ayant de 1 à 6 atomes de carbone.

2. Pseudocéramides selon la revendication 1,
caractérisés en ce que
dans la formule (I) R¹ représente un radical alkyle et/ou alkényle ayant de 12 à 18 atomes de carbone, Y représente de l'oxygène, R² représente de l'hydrogène ou un groupe méthyle, R³ représente un radical hydroxyalkyle ayant 6 atomes de carbone et 5 groupes hydroxyle ou un radical glucosyle et R⁴ représente un radical alkyle et/ou alkényle ayant de 12 à 18 atomes de carbone.

3. Procédé de préparation des pseudocéramides des formules (Ia) ou (Ib) : 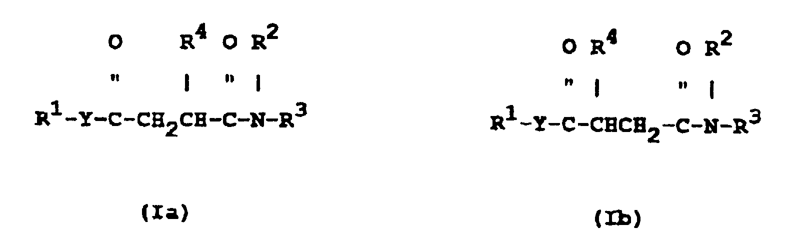 dans lesquelles R¹ représente un radical alkyle et/ou alkényle linéaire ou ramifié, ayant de 6 à 30 atomes de carbone, Y représente de l'oxygène ou un groupe NR⁵, R² représente de l'hydrogène ou un radical alkyle éventuellement substitué par un hydroxy, ayant de 1 à 30 atomes de carbone, R³ représente un radical hydroxyalkyle ayant de 2 à 12 atomes de carbone et de 1 à 10 groupes hydroxyle ou un reste glycosyle, R⁴ représente un radical alkyle et/ou alkényle ayant de 6 à 30 atomes de carbone et R⁵ représente de l'hydrogène ou un radical alkyle éventuellement substitué par un hydroxy ayant de 1 à 6 atomes de carbone,
caractérisé en ce qu'
on transforme un monoester ou un amide d'acide alkysuccinique de formules (IIa) ou (IIb) 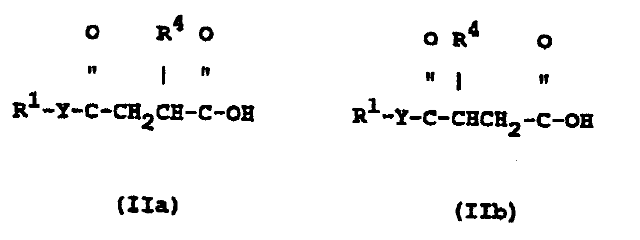 dans lesquelles R¹, R⁴, et Y possèdent les significations mentionnées ci-dessus,
en leur chlorure d'acide ou leurs anhydrides mixtes d'une manière connue en soi à l'aide de dérivés chlorés, et
b) on condense le chlorure d'acide ou l'anhydride mixte en résultant avec des hydroxyalkylamines ou des glucosylamines de formule III : 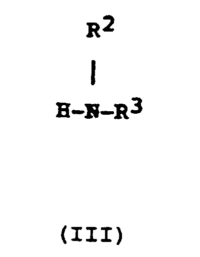 dans laquelle R² et R³ à nouveau possèdent les significations mentionnées ci-dessus.

4. Procédé selon la revendication 3,
caractérisé en ce qu'
on met en oeuvre un monoester d'acide alkyl succinique de formule II dans laquelle R¹ représente un radical alkyle et/ou alkényle ayant de 12 à 18 atomes de carbone, et R⁴ représente un radical alkyle et/ou alkényle ayant de 12 à 18 atomes de carbone.

5. Procédé selon les revendications 3 et 4,
caractérisé en ce qu'
on met en oeuvre comme dérivés chlorés le trichlorure de phosphore, le chlorure de thionyle, ou un éther d'acide chlorocarbonique.

6. Procédé selon les revendications 3 à 5,
caractérisé en ce qu'
on met en oeuvre le monoester ou amide d'acide alkyl succinique et les dérivés chlorés dans le rapport molaire de 1:0,9 à 1:1,1.

7. Procédé selon les revendications 3 à 6,
caractérisé en ce qu'
on met en oeuvre comme hydroxyalkylamines glucosylamines, glucamines, ou le 2,2-bis-(hydroxy méthyl)-2-aminoéthanol.

8. Procédé selon les revendications 3 à 7,
caractérisé en ce qu'
on met en oeuvre les chlorures d'acide ou les anhydrides mixtes et les hydroxyalkylamines ou les glucosylamines dans le rapport molaire 1:0, 9 à 1:1, 1.

9. Utilisation des pseudocéramides selon la revendication 1, comme « lipides de barrière synthétiques » pour la préparation de produits de soins pour la peau.
